# EUROPEAN PATENT APPLICATION

(11) **EP 1 632 496 A1**
(43) Date of publication of application: **08.03.2006**
(21) Application number: 05026136.1
(22) Date of filing: 21.06.1999
(51) Int. Cl.: C07F 9/24, C07F 9/6561, C07D 493/10, C07H 21/00, C07F 9/655

(54) **Reagents and methods for solid phase synthesis and display**

(30) Priority: 22.06.1998 US 102986
(62) Divisional of application: 99250202.1
(71) Applicant: Affymetrix, Inc., Santa Clara, CA 95051 (US)
(72) Inventor: McGall, Glenn H., Mountain View, CA 94043 (US); Diggelmann, Martin, CH-4144 Arlesheim (CH); Barone, Anthony D., San Jose, CA 95125 (US)
(74) Representative: UEXKÜLL & STOLBERG

(57) **Abstract**

New compounds, compositions and methods which find application in solid phase synthesis including the preparation of high-density arrays of diverse polymer sequences such as diverse peptides and oligonucleotides as well as in preparation of arrays of small ligand molecules. The compounds of the present invention are those which are typically referred to as linking groups, linkers or spacers and include unsymmetrical disulfide linking groups, and 1,3-diol derivatives capable of providing a triggered release of an attached compound from a solid support under mild conditions. Additional new compounds are labels which can be incorporated into either the 3' or 5' terminus of a DNA oligomer.

## Description

### BACKGROUND OF THE INVENTION

The present invention relates to the field of solid phase polymer synthesis. More specifically, the invention provides methods and reagents for solid phase synthesis of oligomer arrays and combinatorial chemistry libraries which may be used, for example, in screening studies for determination of binding affinity or other biological activity.

The synthesis of oligomer arrays and combinatorial libraries of small organic molecules has received considerable attention in both academic and industrial research groups. In part, this attention has resulted from the application of such arrays and libraries to drug discovery or screening to obtain sequence information on unsequenced genes or gene fragments. Many of these applications involve the initial preparation of arrays or libraries on a solid support.

The evolution of solid phase synthesis of biological polymers began with the early "Merrifield" solid phase peptide synthesis, described in Merrifield, *J. Am. Chem. Soc.* 85:2149-2154 (1963), incorporated herein by reference for all purposes. Solid-phase synthesis techniques have also been provided for the synthesis of several peptide sequences on, for example, a number of "pins." See *e.g.,* Geysen *et al., J. Immun. Meth.* **102:**259-274 (1987), incorporated herein by reference for all purposes. Other solid-phase techniques involve, for example, synthesis of various peptide sequences on different cellulose disks supported in a column. See Frank and Doring, *Tetrahedron* **44:**6031-6040 (1988), incorporated herein by reference for all purposes. Still other solid-phase techniques are described in U.S. Patent No. 4,728,502 issued to Hamill and WO 90/00626 (Beattie, inventor).

Each of the above techniques produces only a relatively low density array of polymers. For example, the technique described in Geysen *et al.* is limited to producing 96 different polymers on pins spaced in the dimensions of a standard microtiter plate.

Improved methods of forming large arrays of oligonucleotides, peptides and other polymer sequences in a short period of time have been devised. Of particular note, Pirrung *et al.,* U.S. Patent No. 5,143,854 (see also PCT Application No. WO 90/15070) and Fodor *et al.,* PCT Publication No. WO 92/10092, all incorporated herein by reference, disclose methods of forming vast arrays of peptides, oligonucleotides and other polymer sequences using, for example, light-directed synthesis techniques. See also, Fodor *et al., Science,* **251**:767-777 (1991), also incorporated herein by reference for all purposes. These procedures are now referred to as VLSIPS™ procedures.

In the above-referenced Fodor *et al.,* PCT application, an elegant method is described for using a computer-controlled system to direct a VLSIPS™ procedure. Using this approach, one heterogenous array of polymers is converted, through simultaneous coupling at a number of reaction sites, into a different heterogenous array. See, Application Serial Nos. 07/796,243 and 07/980,523, the disclosures of which are incorporated herein for all purposes.

The development of VLSIPS™ technology as described in the above-noted U.S. Patent No. 5,143,854 and PCT patent publication Nos. WO 90/15070 and 92/10092, is considered pioneering technology in the fields of combinatorial synthesis and screening of combinatorial libraries. More recently, patent Application Serial No, 08/082,937, filed June 25, 1993, describes methods for making arrays of oligonucleotide probes that can be used to provide a partial or complete sequence of a target nucleic acid and to detect the presence of a nucleic acid containing a specific oligonucleotide sequence.

### SUMMARY OF THE INVENTION

The present invention provides new compounds, compositions and methods which find application in solid phase synthesis including the preparation of high-density arrays of diverse polymer sequences such as diverse peptides and oligonucleotides as well as in preparation of arrays of small ligand molecules. The compounds of the present invention are those which are typically referred to as linking groups, linkers or spacers.

According to a first aspect of the invention, novel compounds are provided which are unsymmetrical disulfide linking groups. These linking groups allow rapid and mild separation of the synthesized compound from the solid support. Such compounds have the formula: P¹-X¹-(W¹)ₙ-S-S-(W²)ₘ-X²-P². P¹ and P² are each members independently selected from the group consisting of a hydrogen atom, an activating group and a protecting group. X¹ and X² are each independently selected from the group consisting of a bond, -O-, -NH-, -NR- and -CO₂-, wherein R is a lower alkyl group having one to four carbon atoms. W¹ and W² are each independently selected from the group consisting of methylene, oxyethylene and oxypropylene, n and m are each independently integers of from 2 to 12. n and m are not the same when W¹ and W² are the same. P¹ and P² are not both hydrogen atoms.

In another aspect, linking groups are provided which are 1,3-diol derivatives capable of providing a triggered release of an attached compound from a solid support under mild conditions. Such linking groups have the formula 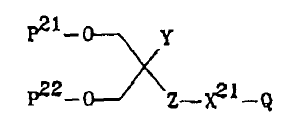 P²¹ and P²² are each protecting groups with the provisos that P²¹ can be removed under conditions which will not remove P²², and P²² can be removed under conditions which will not remove P²¹. X²¹ is a linking moiety selected from the group consisting of an alkylene chain and an aryl group. Y is a substituent selected from the group consisting of -C(=O)R, -S(O)R, -S(O)₂R, -S(O)₂NRR', -CN, -CF₃, -NO₂ and a phenyl ring having one or more substituents selected from the group consisting of halogen, nitro, cyano and trifluoromethyl. Z is a linking moiety selected from the group consisting of -C(=O)-, -S(O)-, -S(O)₂-, -S(O)₂NR-. R and R' are each independently selected from the group consisting of hydrogen, C₁-C₁₂ alkyl and aryl. Q is a phosphate ester-forming group selected from the group consisting of a phosphoramidite and a trialkylammonium H-phosphonate.

According to another aspect of the invention, a novel label is provided which can be incorporated into either the 3' or 5' terminus of a DNA oligomer. The label has the formula 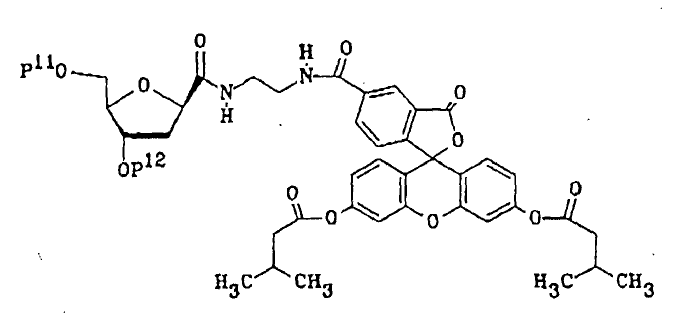 wherein P¹¹ and P¹² are each independently selected from the group consisting of hydrogen, a protecting group, and a phosphodiester-formimg group.

### BRIEF DESCRIPTION OF THE DRAWINGS

Figures 1A, 1B and 1C provide synthesis schemes for the preparation of unsymmetrical disulfide linkages.
Figure 2 provides one synthesis scheme for the 1,3-diol linkages used in the present invention.
Figure 3 illustrates the application of 1,3-diol linkages to solid supports.

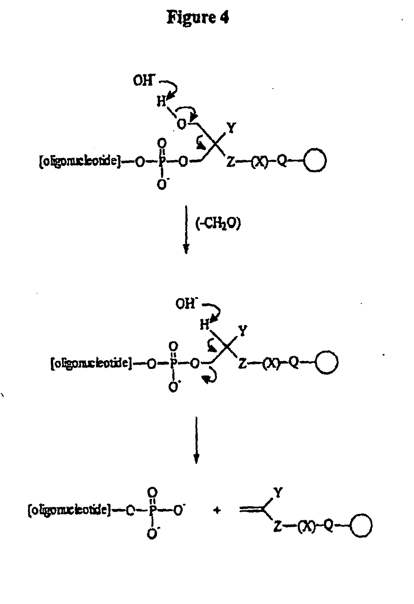

Figure 4 illustrates one possible mechanism for the base-induced release of compounds attached to solid supports via a 1,3-diol linkage.
Figure 5 illustrates a synthesis scheme for the preparation of fluorescein labels for enhanced oligomer detection.

### DETAILED DESCRIPTION OF THE INVENTION

### CONTENTS

### I. Glossary

### II. General

### III. Novel Linking Groups

(a) Unsymmetrical Disulfides
(b) 1,3-Diol Derivatives

### IV. Labels for Enhanced Oligomer Detection

### V. Examples

### VI. Conclusion

### I. Glossary

The following abbreviations are used herein: AcOH, acetic acid; ALLOC, allyloxycarbonyl; BOC, *t*-butyloxycarbonyl; BOP, benzotriazol-1-yloxy-tris(dimethylamino)phosphonium hexafluorophosphate; DIEA, diisopropylethylamine; DMF, dimethylformamide; DMT, dimethoxytrityl; DTT, dithiothreitol; EtOAc, ethyl acetate; FMOC, fluorenylmethyloxycarbonyl; MeNPOC, α-methylnitro-piperonyloxycarbonyl; MeNVOC, α-methylnitroveratryloxycarbonyl; mp, melting point; NVOC, nitroveratryloxycaxbonyl; OBt, hydroxybenzotriazole radical; PHS, phosphate buffered saline; TFA, trifluoroacetic acid; DIPAT, diisopropylammonium tetrazolide; 2-CEBAP; 2-cyanoethyl tetraisopropylphosphorodiamidite; DDZ, α,α-dimethyl-3,5-dimethoxybenzyloxycarbonyl.

The following terms are intended to have the following general meanings as they are used herein:

Chemical terms: As used herein, the term "alkyl" refers to a saturated hydrocarbon radical which may be straight-chain or branched-chain (for example, ethyl, isopropyl, *t*-amyl, or 2,5-dimethylhexyl). When "alkyl" or "alkylene" is used to refer to a linking group or a spacer, it is taken to be a group having two available valences for covalent attachment, for example, -CH₂CH₂- , -CH₂CH₂CH₂-, -CH₂CH₂CH(CH₃)CH₂- and -CH₂(CH₂CH₂)₂CH₂₋Preferred alkyl groups as substituents are those containing 1 to 10 carbon atoms, with those containing 1 to 6 carbon atoms being particularly preferred. Preferred alkyl or alkylene groups as linking groups are those containing 1 to 20 carbon atoms, with those containing 3 to 6 carbon atoms being particularly preferred.

The term "aryl" as used herein, refers to an aromatic substituent which may be a single ring or multiple rings which are fused together, linked covalently or linked to a common group such as an ethylene or methylene moiety. The aromatic rings may each contain heteroatoms, for example, phenyl, naphthyl, biphenyl, diphenylmethyl, 2,2-diphenyl-1-ethyl, thienyl, pyridyl and quinoxalyl, The aryl moieties may also be optionally substituted with halogen atoms, or other groups such as nitro, carboxyl, alkoxy, phenoxy and the like. Additionally, the aryl radicals may be attached to other moieties at any position on the aryl radical which would otherwise be occupied by a hydrogen atom (such as, for example, 2-pyridyl, 3-pyridyl and 4-pyridyl). As used herein, the term "aralkyl'' refers to an alkyl group bearing an aryl substituent (for example, benzyl, phenylethyl, 3-(4-nitrophenyl)propyl, and the like).

The term "protecting group" as used herein, refers to any of the groups which are designed to block one reactive site in a molecule while a chemical reaction is carried out at another reactive site. More particularly, the protecting groups used herein can be any of those groups described in Greene, *et al., Protective Groups In Organic Chemistry,* 2nd Ed., John Wiley & Sons, New York, NY, 1991, incorporated herein by reference. The proper selection of protecting groups for a particular synthesis will be governed by the overall methods employed in the synthesis. For example, in "light-directed" synthesis, discussed below, the protecting groups will be photolabile protecting groups such as dimethoxybenzoin, NVOC, MeNPOC, and those disclosed in co-pending Application PCT/US93/10162 (filed October 22, 1993), incorporated herein by reference. In other methods, protecting groups may be removed by chemical methods and include groups such as FMOC, DMT and others known to those of skill in the art.

The term "activating agent" refers to those groups which, when attached to a particular functional group or reactive site, render that site more reactive toward covalent bond formation with a second functional group or reactive site. For example, the group of activating groups which are useful for a carboxylic acid include simple ester groups and anhydrides. The ester groups include alkyl, aryl and alkenyl esters and in particular such groups as 4-nitrophenyl, N-hydroxylsuccinimide and pentafluorophenol. Other activating groups will include phosphodiester-forming groups such as phosphoramidates, phosphite-triesters, phosphotriesters, and H-phosphonates. Still other activating agents are known to those of skill in the art.

Monomer: A monomer is a member of the set of small molecules which are or can be joined together to form a polymer or a compound composed of two or more members. The set of monomers includes but is not restricted to, for example, the set of common L-amino acids, the set of D-amino acids, the set of synthetic and/or natural amino acids, the set of nucleotides and the set of pentoses and hexoses. The particular ordering of monomers within a polymer is referred to herein as the "sequence" of the polymer. As used herein, monomers refers to any member of a basis set for synthesis of a polymer. For example, dimers of the 20 naturally occurring L-amino acids form a basis set of 400 monomers for synthesis of polypeptides. Different basis sets of monomers may be used at successive steps in the synthesis of a polymer. Furthermore, each of the sets may include protected members which are modified after synthesis. The invention is described herein primarily with regard to the preparation of molecules containing sequences of monomers such as amino acids, but could readily be applied in the preparation of other polymers. Such polymers include, for example, both linear and cyclic polymers of nucleic acids, polysaccharides, phospholipids, and peptides having either α-, β-, or ω-amino acids, heteropolymers in which a known drug is covalently bound to any of the above, polynucleotides, polyurethanes, polyesters, polycarbonates, polyureas, polyamides, polyethyleneimines, polyarylene sulfides, polysiloxanes, polyimides, polyacetates, or other polymers which will be apparent upon review of this disclosure. Such polymers are "diverse" when polymers having different monomer sequences are formed at different predefined regions of a substrate. Methods of cyclization and polymer reversal of polymers are disclosed in copending application USSN 07/978940 which is a CIP of U.S. Patent No. 5,242,974 entitled "POLYMER REVERSAL ON SOLID SURFACES," incorporated herein by reference for all purposes.

Polymer: A polymer is formed by covalent linkage of at least two monomer units. Polymers can incorporate any number of monomer units. Examples of polymers include oligonucleotides, peptides and carbohydrates.

Oligonucleotide: An oligonucleotide can be DNA or RNA (i.e, a nucleic acid), and single- or double-stranded. Oligonucleotides can be naturally occurring or synthetic. The segments are usually between 2 and 100 bases, but can be of any length. Lengths between 5-10, 5-20, 10-20, 10-50, 20-50 or 20-100 bases are common.

Peptide: A peptide is a polymer in which the monomers are amino acids and are joined together through amide bonds, alternatively referred to as a polypeptide. When the amino acids are α-amino acids, either the L-optical isomer or the D-optical isomer may be used. Additionally, unnatural amino acids, for example, β-alanine, phenylglycine and homoarginine are also meant to be included. Peptides are two or more amino acid monomers long, can be of any length and are often more than 20 amino acid monomers long.

Substrate: A material having a rigid or semi-rigid surface. In many embodiments, at least one surface of the substrate will be substantially flat, although in some embodiments it may be desirable to physically separate synthesis regions for different polymers with, for example, wells, raised regions, etched trenches, or the like. In some embodiments, the substrate itself contains wells, trenches, flow through regions, etc. which form all or part of the synthesis regions. According to other embodiments, small beads may be provided on the surface, and compounds synthesized thereon may be released upon completion of the synthesis.

Channel Block: A material having a plurality of grooves or recessed regions on a surface thereof. The grooves or recessed regions may take on a variety of geometric configurations, including but not limited to stripes, circles, serpentine paths, or the like. Channel blocks may be prepared in a variety of manners, including etching silicon blocks, molding or pressing polymers, etc.

Predefined Region: A predefined region is a localized area on a substrate which is, was, or is intended to be used for formation of a selected polymer and is otherwise referred to herein in the alternative as "reaction" region, a "selected" region, or simply a "region." The predefined region may have any convenient shape, e.g., circular, rectangular, elliptical, wedge-shaped, etc. In some embodiments, a predefined region and, therefore, the area upon which each distinct polymer sequence is synthesized is smaller than about 1 cm², more preferably less than 1 mm², and still more preferably less than 0.5 mm², In most preferred embodiments the regions have an area less than about 10,000 µm² or, more preferably, less than 100 *µ*m². Within these regions, the polymer synthesized therein is preferably synthesized in a substantially pure form. Additionally, multiple copies of the polymer will typically be synthesized within any preselected region. The number of copies can be in the thousands to the millions.

### II. General

The compounds, compositions and methods of the present invention can be used in a number of solid phase synthesis applications, including light-directed methods, flow channel and spotting methods, pin-based methods and bead-based methods.

### Light-Directed Methods

"Light-directed" methods (which are one technique in a family of methods known as VLSIPS™ methods) are described in U.S. Patent No, 5,143,854, previously incorporated by reference. The light directed methods discussed in the '854 patent involve activating predefined regions of a substrate or solid support and then contacting the substrate with a preselected monomer solution. The predefined regions can be activated with a light source, typically shown through a mask (much in the manner of photolithography techniques used in integrated circuit fabrication). Other regions of the substrate remain inactive because they are blocked by the mask from illumination and remain chemically protected. Thus, a light pattern defines which regions of the substrate react with a given monomer. By repeatedly activating different sets of predefined regions and contacting different monomer solutions with the substrate, a diverse array of polymers is produced on the substrate. Of course, other steps such as washing unreacted monomer solution from the substrate can be used as necessary.

### Flow Channel or Spotting Methods

Additional methods applicable to library synthesis on a single substrate are described in co-pending Applications Serial No. 07/980,523, filed November 20, 1992, and 07/796,243, filed November 22, 1991, incorporated herein by reference for all purposes. In the methods disclosed in these applications, reagents are delivered to the substrate by either (1) flowing within a channel defined on predefined regions or (2) "spotting" on predefined regions. However, other approaches, as well as combinations of spotting and flowing, may be employed. In each instance, certain activated regions of the substrate are mechanically separated from other regions when the monomer solutions are delivered to the various reaction sites.

A typical "flow channel" method applied to the compounds and libraries of the present invention can generally be described as follows- Diverse polymer sequences are synthesized at selected regions of a substrate or solid support by forming flow channels on a surface of the substrate through which appropriate reagents flow or in which appropriate reagents are placed. For example, assume a monomer "A" is to be bound to the substrate in a first group of selected regions. If necessary, all or part of the surface of the substrate in all or a part of the selected regions is activated for binding by, for example, flowing appropriate reagents through all or some of the channels, or by washing the entire substrate with appropriate reagents. After placement of a channel block on the surface of the substrate, a reagent having the monomer A flows through or is placed in all or some of the channel(s). The channels provide fluid contact to the first selected regions, thereby binding the monomer A on the substrate directly or indirectly (via a spacer) in the first selected regions.

Thereafter, a monomer B is coupled to second selected regions, some of which may be included among the first selected regions. The second selected regions will be in fluid contact with a second flow channel(s) through translation, rotation, or replacement of the channel block on the surface of the substrate; through opening or closing a selected valve; or through deposition of a layer of chemical or photoresist. If necessary, a step is performed for activating at least the second regions. Thereafter, the monomer B is flowed through or placed in the second flow channel(s), binding monomer B at the second selected locations. In this particular example, the resulting sequences bound to the substrate at this stage of processing will be, for example, A, B, and AB. The process is repeated to form a vast array of sequences of desired length at known locations on the substrate.

After the substrate is activated, monomer A can be flowed through some of the channels, monomer B can be flowed through other channels, a monomer C can be flowed through still other channels, etc. In this manner, many or all of the reaction regions are reacted with a monomer before the channel block must be moved or the substrate must be washed and/or reactivated. By making use of many or all of the available reaction regions simultaneously, the number of washing and activation steps can be minimized.

One of skill in the art will recognize that there are alternative methods of forming channels or otherwise protecting a portion of the surface of the substrate. For example, according to some embodiments, a protective coating such as a hydrophilic or hydrophobic coating (depending upon the nature of the solvent) is utilized over portions of the substrate to be protected, sometimes in combination with materials that facilitate wetting by the reactant solution in other regions. In this manner, the flowing solutions are further prevented from passing outside of their designated flow paths.

The "spotting" methods of preparing compounds and libraries of the present invention can be implemented in much the same manner as the flow channel methods. For example, a monomer A can be delivered to and coupled with a first group of reaction regions which have been appropriately activated. Thereafter, a monomer B can be delivered to and reacted with a second group of activated reaction regions. Unlike the flow channel embodiments described above, reactants are delivered by directly depositing (rather than flowing) relatively small quantities of them in selected regions. In some steps, of course, the entire substrate surface can be sprayed or otherwise coated with a solution. In preferred embodiments, a dispenser moves from region to region, depositing only as much monomer as necessary at each stop. Typical dispensers include a micropipette to deliver the monomer solution to the substrate and a robotic system to control the position of the micropipette with respect to the substrate, or an ink-jet printer. In other embodiments, the dispenser includes a series of tubes, a manifold, an array of pipettes, or the like so that various reagents can be delivered to the reaction regions simultaneously.

### Pin-Based Methods

Another method which is useful for the preparation of compounds and libraries of the present invention involves "pin based synthesis." This method is described in detail in U.S. Patent No, 5,288,514, previously incorporated herein by reference. The method utilizes a substrate having a plurality of pins or other extensions. The pins are each inserted simultaneously into individual reagent containers in a tray. In a common embodiment, an array of 96 pins/containers is utilized.

Each tray is filled with a particular reagent for coupling in a particular chemical reaction on an individual pin. Accordingly, the trays will often contain different reagents. Since the chemistry disclosed herein has been established such that a relatively similar set of reaction conditions may be utilized to perform each of the reactions, it becomes possible to conduct multiple chemical coupling steps simultaneously. In the first step of the process the invention provides for the use of substrate(s) on which the chemical coupling steps are conducted. The substrate is optionally provided with a spacer having active sites. In the particular case of oligonucleotides, for example, the spacer may be selected from a wide variety of molecules which can be used in organic environments associated with synthesis as well as aqueous environments associated with binding studies. Examples of suitable spacers are polyethyleneglycols, dicarboxylic acids, polyamines and alkylenes, substituted with, for example, methoxy and ethoxy groups. Additionally, the spacers will have an active site on the distal end. The active sites are optionally protected initially by protecting groups. Among a wide variety of protecting groups which are useful are FMOC, BOC, t-butyl esters, t-butyl ethers, and the like. Various exemplary protecting groups are described in, for example, Atherton *et al. , Solid Phase Peptide Synthesis,* IRL Press (1989), incorporated herein by reference In some embodiments, the spacer may provide for a cleavable function by way of, for example, exposure to acid or base.

### Bead Based Methods

Yet another method which is useful for synthesis of polymers and small ligand molecules on a solid support "bead based synthesis." A general approach for bead based synthesis is described copending Application Serial Nos. 07/762,522 (filed September 18, 1991); 07/946,239 (filed September 16, 1992); 08/146,886 (filed November 2, 1993); 07/876,792 (filed April 29, 1992) and PCT/US93/04145 (filed April 28, 1993), the disclosures of which are incorporated herein by reference.

For the synthesis of molecules such as oligonucleotides on beads, a large plurality of beads are suspended in a suitable carrier (such as water) in a container. The beads are provided with optional spacer molecules having an active site. The active site is protected by an optional protecting group.

In a first step of the synthesis, the beads are divided for coupling into a plurality of containers. For the purposes of this brief description, the number of containers will be limited to three, and the monomers denoted as A, B, C, D, E, and F, The protecting groups are then removed and a first portion of the molecule to be synthesized is added to each of the three containers (*i.e.,* A is added to container 1, B is added to container 2 and C is added to container 3).

Thereafter, the various beads are appropriately washed of excess reagents, and remixed in one container. Again, it will be recognized that by virtue of the large number of beads utilized at the outset, there will similarly be a large number of beads randomly dispersed in the container, each having a particular first portion of the monomer to be synthesized on a surface thereof.

Thereafter, the various beads are again divided for coupling in another group of three containers. The beads in the first container are deprotected and exposed to a second monomer (D), while the beads in the second and third containers are coupled to molecule portions E and F respectively. Accordingly, molecules AD, BD, and CD will be present in the first container, while AE, BE, and CE will be present in the second container, and molecules AF, BF, and CF will be present in the third container. Each bead, however, will have only a single type of molecule on its surface. Thus, all of the possible molecules formed from the first portions A, B, C, and the second portions D, E, and F have been formed.

The beads are then recombined into one container and additional steps such as are conducted to complete the synthesis of the polymer molecules. In a preferred embodiment, the beads are tagged with an identifying tag which is unique to the particular compound which is present on each bead. A complete description of identifier tags for use in synthetic libraries is provided in co-pending Application Serial No. 08/146,886 (filed November 2, 1993) previously incorporated by reference for all purposes.

### Utilities of Chemical Libraries

The advent of methods for the synthesis of diverse chemical compounds on solid supports has resulted in the genesis of a multitude of diagnostic applications for such chemical libraries. A number of these diagnostic applications involve contacting a sample with a solid support, or chip, having multiple attached biological polymers such as peptides and oligonucleotides, or other small ligand molecules synthesized from building blocks in a stepwise fashion, in order to identify any species which specifically binds to one or more of the attached polymers or small ligand molecules.

For example, patent Application Serial No. 08/082,937, filed June 25, 1993, describes methods for making arrays of oligonucleotide probes that can be used to provide the complete sequence of a target nucleic acid and to detect the presence of a nucleic acid containing a specific oligonucleotide sequence. Patent application Serial No. 08/327,687, filed October 24, 1994, now U.S. Patent No. 5,556,752, describes methods of making arrays of unimolecular, double-stranded oligonucleotides which can be used in diagnostic applications involving protein/DNA binding interactions such as those associated with the p53 protein and the genes contributing to a number of cancer conditions. Arrays of double-stranded oligonucleotides can also be used to screen for new drugs having particular binding affinities. The linking groups and labels provided herein are useful in each of these library applications, as well as others now known in the literature.

### III. Novel Linking Groups

In one aspect, the present invention provides novel linking groups which can facilitate oligomer or small molecule synthesis on a solid support and which can provide rapid release from the support under very mild conditions. Some of the linking groups are unsymmetrical disulfide linking groups and other linking groups are derivatives of 1,3-diols which provide an effective "trigger" for the mild removal of a synthesized compound from a solid support.

### (a) Unsymmetrical Disulfides

One group of unsymmetrical disulfide compounds which can be used as linking groups are represented by the formula:

P¹-X¹-(W¹)ₙ-S-S-(W²)ₘ-X²P² (I)

In this formula, P¹ and P² are each independently a hydrogen atom, an activating group (*e.g.,* a phosphodiester-forming group) or a selectively removable protecting group. However, P¹ and P² will not both be hydrogen atoms. The symbols X¹ and X² each independently represent a bond, -O-, -NH-, -NR- and -CO₂-, in which R is an alkyl group having one to four carbon atoms. The symbols W¹ and W² each independently represent a methylene group (-CH₂-), an oxyethylene group (-OCH₂CH₂- or -CH₂CH₂O-), an oxypropylene group (*e.g*., -OCH₂CH₂CH₂-, -OCH₂CH(CH₃)- or -CH(CH₃)CH₂O-), and the like. The letters n and m each independently represent integers of from 2 to 12, with the proviso that n and m are not the same integer when W¹ and W² are identical. Preferably, the letters n and m represent integers of from 3 to 8. All numerical ranges in this application are meant to be inclusive of their upper and lower limits.

In one group of embodiments, P¹ is a photocleavable protecting group, preferably an NVOC, MeNPOC, Dimethoxybenzoinyl, or α,α-dimethyl-3,5-dimethoxybenzyloxy-carbonyl (DDZ). More preferably, P¹ is a MeNPOC protecting group. In another group of embodiments, P¹ is DMT, FMOC or BOC, more preferably DMT.

The linking groups of formula (I) are useful as a 3'-end cleavable linking group in any solid phase synthesis of oligonucleotides. When used for this solid phase preparation of oligonucleotides, P² is preferably an activating group such as a phosphoramidite or other functionally equivalent group commonly used in solid phase oligonucleotide synthesis. Detailed descriptions of the procedures for solid phase synthesis of oligonucleotides by phosphiee-triester, phosphotriester, and H-phosphonate chemistries are widely available. See, for example, Itakura, U.S. Pat. No. 4,401,796; Caruthers, *et al.,* U.S- Pat. Nos. 4,458,066 and 4,500,707; Beaucage, *et al., Tetrahedron Lett.,* **22:**1859-1862 (1981); Matteucci, *et al., J. Am. Chem. Soc.,* **103**:3185-3191 (1981); Caruthers, *et al., Genetic Engineering,* **4**:1-17 (1982); Jones, chapter 2, Atkinson, *et al,,* chapter 3, and Sproat, *et al. ,* chapter 4, in *Oligonucleotide Synthesis: A Practical Approach,* Gait (ed.), IRL Press, Washington D.C. (1984); Froehler, *et al., Tetrahedron Lett.,* 27:469-472 (1986); Froehler, *et al., Nucleic Acids Res.,* **14**:5399-5407 (1986); Sinha, *et al. Tetrahedron Lett.,* **24**:5843-5846 (1983); and Sinha, *et al., Nucl. Acids Res.,* **12**:4539-4557 (1984) which are incorporated herein by reference. In these embodiments X² is preferably -O-.

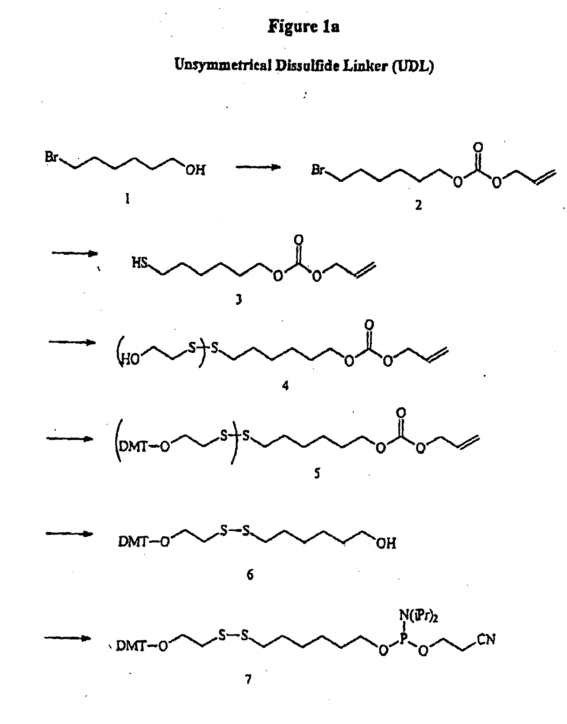

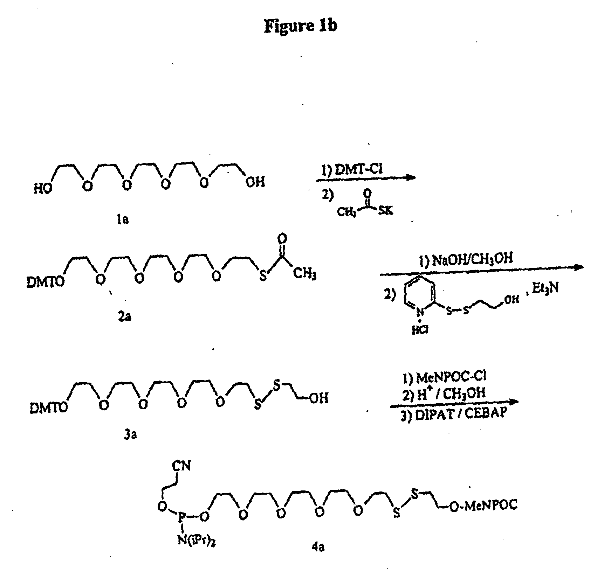

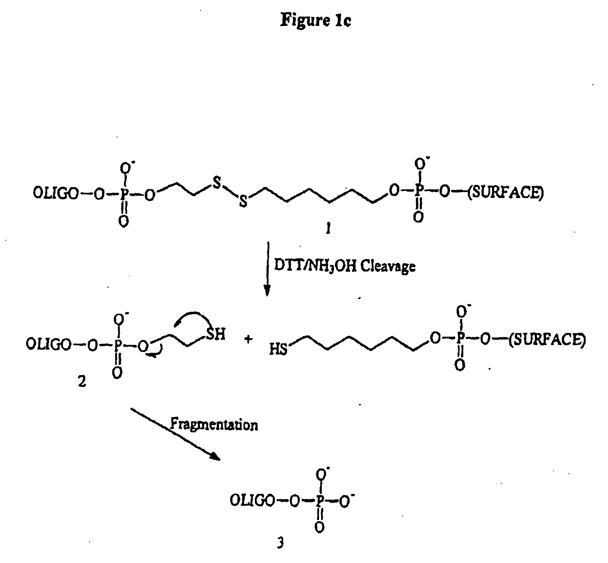

The unsymmetrical disulfide linking groups of the present invention can be prepared by methods which are known to those of skill in the an. Figures 1a and 1b provide synthesis schemes for preparation of the linkers. According to Figure 1a, commercially available 6-bromohexanol (Aldrich Chemical Company, Milwaukee, Wisconsin, USA) can be treated with allyl chloroformate in pyridine to produce the allyl carbonate (2 in Figure 1a). The terminal bromide can be converted to a thiol upon treatment with sodium hydrogen sulfide in THF/H₂O at pH 7. Disulfide functionality can then be introduced upon reaction of thiol 4 with 2-pyridyl-2-hydroxyethyl disulfide in THF and triethylamine. Protection of the terminal hydroxyl group is accomplished with DMT chloride in pyridine to provide 6. Conversion of 6 to 8 can be achieved by removal of the allyl carbonate (catalytic K₂CO₃ in MeOH) and treatment of the resultant hydroxyl group with DiPAT and BAP. As can be seen, this methodology provides a linking group of the formula above in which P¹ is DMT, X¹ is -O-, n is 2, m is 6, X² is -O- and P² is phosphoramidite.

As shown in Fig. 1C, when N=2, the disulfide bond of 1 cleaves under netural or basic conditions in the presence of DTT to give an oligonucleotide 2, which possess the 2-mercaptoehtyl phosphate ester at the 3'-end. This ester fragments have been observed efficiently to produce the 3-phosphorylated oligonucleotide 3. This product has been shown to be identical to that produced by the base catalyzed cleavage of oligonucleotides tethered to the surface via the known Phosphate-ON reagent (Glen Research). The unsymmetrical disulfie linker when N=2 is preferred when it is desirable to cleave from the surface and analyze the oligonucleotides by HPLC, since the resultant oligonucleotides do not possess a 3'-thiol appendage. In the cases where N > 2, the mercpatoalkyl esters should be more stable and the cleaved oligonucleotides retain the corresponding thiol appendage. This makes subsequent analysis of the cleaved DNA diffciult because of oxitation of the thiol group.

The steps just described can be suitably modified by one of skill in the art to prepare a number of related analogs based upon the availability of bromo alcohols (1 as starting material) and 2-pyridyl disulfide alcohols. According to Figure 1b, pentaethylene glycol (1a) is first protected with DMT-Cl, then converted to a mono thioester (2a) with potassium thioacetate. Cleavage of the acetyl group with base, and reaction of the resultant thiol functionality with 2-pyridyl 3-hydroxyethyl disulfide provides a mono-protected unsymmetrical disulfide linker (3a). Protection of the hydroxyl group with MeNPOC-Cl, followed by removal of the DMT protecting group and conversion of the liberated hydroxyl group to a phosphoramidite, provides linker (4a) which is useful in automated oligomer synthesizers.

In a related aspect, the present invention provides modified substrates which are useful in the solid phase synthesis of oligonucleotides as well as small ligand molecules. The substrates are derivatized with the unsymmetrical disulfide linking groups described above and are represented by the formula:

A¹-B¹-L¹ (II)

in which A¹ is a solid substrate, B¹ is a bond or a spacer and L¹ is an unsymmetrical disulfide linking group having the formula:

P¹-X¹-(W¹)ₙ-S-S-(W¹)ₘ-X²- (IIa)

In formula (IIa), the symbol P¹ represents a hydrogen or a protecting group. The symbols X¹ and X² are each independently a bond, -O-, -NH-, -NR- and -CO₂-, wherein R is a lower alkyl group having one to four carbon atoms. The symbols W¹ and W² are as described above. The letters n and m represent, as above, integers of from 2 to 12 with the understanding that n and m are not the same when W¹ and W² are identical,

In this aspect of the invention, the solid substrates may be biological, nonbiological, organic, inorganic, or a combination of any of these, existing as particles, strands, precipitates, gels, sheets, tubing, spheres, containers, capillaries, pads, slices, films, plates, slides, *etc.* The solid substrate is preferably flat but may take on alternative surface configurations. For example, the solid substrate may contain raised or depressed regions on which synthesis takes place. In some embodiments, the solid substrate will be chosen to provide appropriate light-absorbing characteristics. For example, the substrate may be a polymerized Langmuir Blodgett film, functionalized glass, Si, Ge, GaAs, GaP, SiO₂, SiN₄, modified silicon, or any one of a variety of gels or polymers such as (poly)tetrafluoroethylene, (poly)vinylidendifluoride, polystyrene, polycarbonate, or combinations thereof. Other suitable solid substrate materials will be readily apparent to those of skill in the art, Preferably, the surface of the solid substrate will contain reactive groups, which are carboxyl, amino, hydroxyl, thiol, or the like. More preferably, the surface will be optically transparent and will have surface Si-OH functionalities, such as are found on silica surfaces.

For those embodiments in which B¹ is a spacer, it will be attached to the solid substrate via carbon-carbon bonds using, for example, substrates having (poly)trifluorochloroethylene surfaces, or more preferably, by siloxane bonds (using, for example, glass or silicon oxide as the solid substrate). Siloxane bonds with the surface of the substrate are formed in one embodiment via reactions of derivatization reagents bearing trichlorosilyl or trialkoxysilyl groups.

The particular spacer can be selected based upon its hydrophilic or hydrophobic properties to improve presentation of an attached oligomer or compound to certain receptors, proteins or drugs. Prior to attachment to the solid substrate the spacer will have a substrate attaching group at one end, and a reactive site at the other end. The reactive site will be a group which is appropriate for attachment to the linking group, L¹. For example, groups appropriate for attachment to a silica surface would include trichlorosilyl and trialkoxysilyl functional groups. Groups which are suitable for attachment to a linking group include amine, hydroxyl, thiol, carboxylic acid, ester, amide, isocyanate and isothiocyanate. Preferred spacers include aminoalkyltrialkoxysilanes, hydroxyalkyltrialkoxysilanes, polyethyleneglycols, polyethyleneimine, polyacrylamide, polyvinylalcohol and combinations thereof.

The unsymmetrical disulfide linking groups used in the present modified substrates are represented by radicals of the formula:

P¹-X¹-(W¹)ₙ-S-S-(W²)ₘ X²- (IIa)

in which X¹, W¹, W², n, m, X² and P¹ have the meanings as provided above. In preferred embodiments, n and m are integers of from 3 to 8, and W¹ and W² are either methylene groups or oxyethylene groups. In other preferred embodiments, X¹ and X² are each independently -O- or -NH-, most preferably X¹ and X² are both -O-. In still other preferred embodiments, P¹ is a protecting group, more preferably a DMT group.

Attachment of the linking group L¹ to a functional group on the solid support or to a reactive site on a spacer can be accomplished using standard chemical methods. For example, when X² is oxygen, linkage to a carboxylic acid or activated carboxylic acid can be made using standard ester-forming reactions. Alternatively, the X² group (derived from a hydroxyl group) can be reacted with support bound isocyanate groups to form carbamate linkages. In other embodiments, X² will be attached to a solid support via a phosphodiester or phosphotriester linkage. In these embodiments, the attachment will typically occur via a phosphoamidite or other phosphodiester or triester-forming group initially present on the unsymmetrical disulfide linking group.

In yet another aspect, the present invention provides methods for the preparation of small ligand molecules or oligonucleotides on a solid support. The methods typically comprise:
(a) contacting a solid support with an unsymmetrical disulfide linking group of formula:

   P¹-X¹-(W¹)ₙ-S-S-(W²)ₘ-X²-P² **(IIb)**

   in which P¹ is a protecting group; P² is a phosphoramidite or other phosphodiester-forming group; X¹ and X² are each independently a bond, ―O―, -NH-, ―NR― or ―CO₂―, wherein R is a lower alkyl group having one to four carbon atoms; the symbols W¹ and W² each independently represent a methylene group (―CH₂―), an oxyethylene group (-OCH₂CH₂- or -CH₂CH₂O-), an oxypropylene group (*e.g*., -OCH₂CH₂CH₂-, -OCH₂CH(CH₃)- or -CH(CH₃)CH₂O-), and the like; and n and m are each independently integers of from 2 to 12 with the proviso that n and m are not the same when W¹ and W² are the same, under conditions sufficient to produce a derivatized solid support having attached unsymmetrical disulfide linking groups suitably protected with protecting groups;
(b) optionally removing the protecting groups from the derivatized solid support to provide a derivatized solid support having unsymmetrical disulfide linking groups with synthesis initiation sites; and
(c) coupling the oligonucleotides or small ligand molecules to the synthesis initiation sites on the derivatized solid support to produce a solid support having attached small ligand molecules or oligonucleotides which are removable therefrom upon application of a disulfide cleaving reagent.

Attaching the unsymmetrical disulfide linking group radical to the solid support can generally be carried out by standard chemical methods such as those described above. In a preferred embodiment, X² is -0- and P² is a phosphoramidite. In this embodiment the unsymmetrical disulfide linking group can be reacted with a solid support having available hydroxyl groups using standard nucleic acid synthesis techniques. The product is a solid support having unsymmetrical disulfide linking groups which are attached via a phosphodiester linkage. The distal end of the linking group (that end furthest removed from the solid support will be either a synthesis initiation site or a protected synthesis initiation site.

When present, the optional protecting groups can be removed using well known methods which will not interfere with molecules or groups present on the support. In preferred embodiments, the removal of protecting groups can be carried out at particular predefined regions on the support using light and photolithographic masks, or flow channel or spotting techniques with appropriate removal reagents. The removal of the protecting groups provides a solid support having attached unsymmetrical disulfide linkages and synthesis initiation sites.

The preparative methods then continue with the coupling of monomers, molecules or components of molecules to the synthesis initiation sites. Again, the chemistry of coupling follows standard synthesis methodology known to those of skill in the art.

Preferred embodiments for this aspect of the invention are generally as described above for the related compounds and modified substrates. In a particularly preferred embodiment, -(W¹)ₙ- is -CH₂CH₂-.

### (b) 1,3-Diol Linking Groups

In another aspect, the present invention provides novel cleavable linking groups which are derivatives of 1,3-diols. These linking groups provide a selectively cleavable linkage between an oligomer or small molecule and a solid support. The linkage is stable to conditions of oligonucleotide synthesis, deprotection steps, and hybridization. The 1,3-diol linkers of the present invention can be represented by the formula: 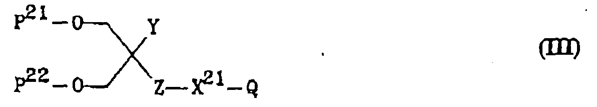 In this formula, P²¹ and P²² are selectively removable protecting groups, Y is an electron-withdrawing substituent, Z is an electron-withdrawing linking moiety, X is a divalent radical derived from an alkyl, aryl or aralkyl group, and Q is a phosphate ester, phosphoramidite or trialkylammonium H-phosphoate moiety.

In one group of embodiments, the symbol Y represents an electron-withdrawing substituent which can be nitro (-NO₂), cyano (-CN). trifluoromethyl (-CF₃), or a substituted aryl group in which the substituents on the aromatic ring are halogen, nitro, cyano, trifluoromethyl or combinations thereof. Y can also be an acyl (-COR'), sulfinyl (-SOR'), sulfonyl (-SO₂R') or sulfonamide group (-SO₂NR'₂) in which the R' portion is an alkyl or aryl group of from 1 to 8 carbon atoms such that the size of the R' portion does not interfere with oligomer synthesis. In, preferred embodiments, Y is a cyano group or an acyl group, more preferably an acetyl group.

The symbol Z represents an electron-withdrawing connector which is ―CO―, -CONH-, -CONR"-, -SO-, -SO₂- or -SO₂NR"- in which the R" portion is an alkyl or aryl group of from 1 to 6 carbon atoms such that the size of the R" portion does not interfere with oligomer or small ligand molecule synthesis. In preferred embodiments, Z is -CO-, -CONH- or -CONR"-. In particularly preferred embodiments, Z is -CONH-.

The symbol Q represents a phosphorus-containing ester group which is capable of facilitating formation of phosphodiester linkages or phosphotriester linkages. Examples of suitable phosphorus-containing ester groups are phosphoramidites (*e.g*., O-(2-cyanoethyl)-N,N-dialkylphosphoramidite) and trialkylammonium H-phosphonates. Other suitable phosphorus-containing groups, are those which have been described with respect to P²for the unsymmetrical disulfide linking groups above.

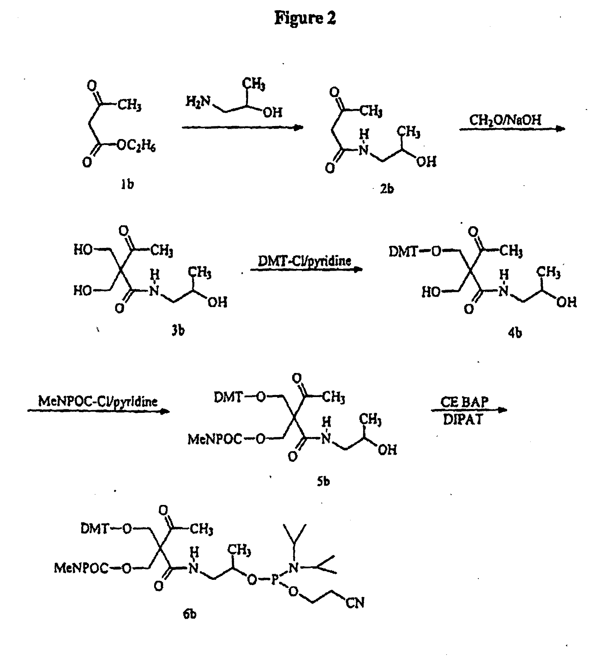

Synthesis of the 1,3-diol linkages can be accomplished via reactions as outlined in Figure 2. According to this synthetic scheme, a 1,3-dicarbonyl compound (ethyl acetoacetate **1b**) is converted to an amide 2b using 1-amino-2-propanol. The reactive methylene center is alkylated with formaldehyde and base under conditions in which two hydroxymethyl groups become attached to the activated center to form a 1,3-diol 3b. Protection of one of the primary hydroxy functional groups is carried out with DMT-Cl in pyridine. The remaining primary hydroxy functional group is protected with a second group (*e.g*., MeNPOC, using McNPOC-Cl in pyridine). Conversion of the remaining hydroxy functional group to a phosphorus-containing ester group can be accomplished using, for example, [CEBAP and DIPAT]) and a suitable base to provide the target 1,3-diol linking group 6b.

One of skill in the art will understand that the reaction scheme presented in Figure 2 can be modified for use with a variety of 1,3-dicarbonyl starting materials, or other di-activated methylene compounds. The amino alcohol used in the first reaction step can also be substituted with alternative amino alcohols. Further, the primary hydroxy functional groups can be protected with any of a variety of protecting groups which are selectively removable in the presence of the other protecting group. Still further, the modification of the secondary hydroxyl group to a phosphoramidite or other phosphorus-ester forming group can be accomplished with any of the reagents used in oligonucleotide synthesis and known to those of skill in the art.

In a related aspect, the present invention provides modified substrates which are useful in the solid phase synthesis of oligonucleotides as well as small ligand molecules. The substrates are derivatized with the 1,3-diol linking groups described above and are represented by the formula:

A²-B²-L² (IV)

in which A² is a solid substrate, B² is a bond or a spacer and L² is a 1,3-diol linking group having the formula: 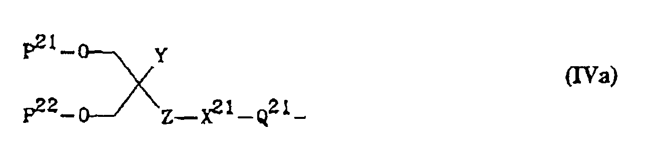 In formula (IVa), the symbols P²¹ and P²² each independently represent a protecting group. The symbols X²¹, Y and Z have the meaning provided above for formula (III). The symbol Q²¹ represents a phosphodiester or phosphotriester linkage.

In this aspect of the invention, the solid substrates (A²) and spacer (B²) are as described above for A¹ and B¹.

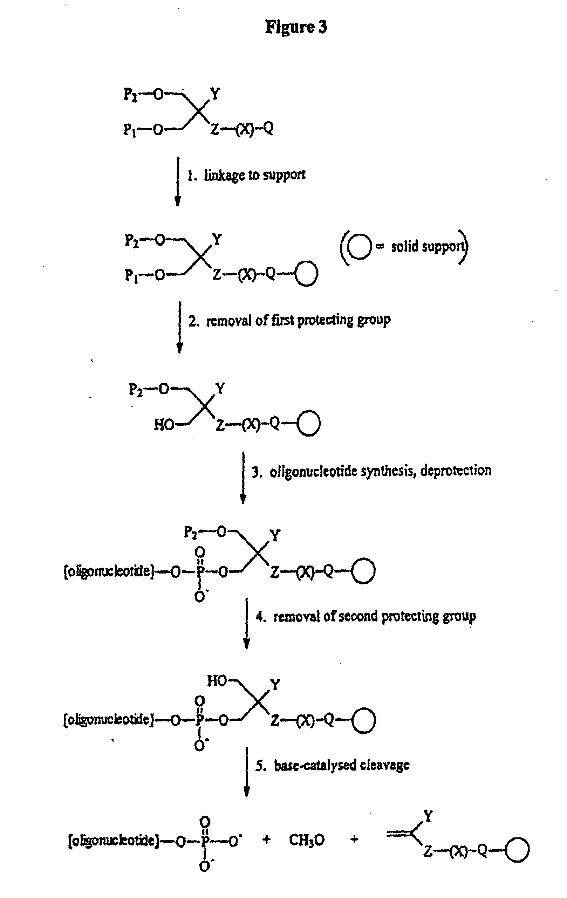

Attachment of the linking group L² to a functional group on the solid support or to a reactive site on a spacer can be accomplished using standard chemical methods (See Figure 3). For example, when Q²¹ is a phosphodiester, the linking group can be attached to the solid support using methods typically used for solid phase synthesis of oligonucleotides (*e.g.,* via phosphoramidite chemistry). Figure 3 further provides an illustration of the use of a modified support having a 1,3-diol linkage according to the present invention. In this figure, the linking group is first attached to the support as described above. One of the two protecting groups (P²¹ or P²²) is then removed to provide a hydroxyl group as a synthesis initiation site. An oligonucleotide (or other small ligand molecule) can then be synthesized on the initiation site using methods described above. Release of the newly prepared oligonucleotide or other molecule from the solid support can be accomplished by removal of the second protecting group and treatment with base as indicated.

In view of the above, the present invention further provides a method of synthesizing small ligand molecules or oligonucleotides on a solid support having optional spacers, the small ligand molecules being removable therefrom upon treatment with a base. The method comprises:
(a) contacting a solid support with a 1,3-diol linking group of formula: 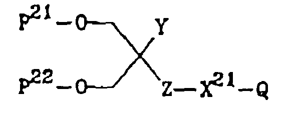 wherein P²¹ and P²² are each protecting groups with the provisos that P²¹ can be removed under conditions which will not remove P²²_{;} and P²² can be removed under conditions which will not remove P²¹ X²¹ is a linking moiety selected from the group consisting of an alkylene chain and an aryl group; Y is a substituent selected from the group consisting of -C(=O)R, -S(O)R, -S(O)₂R, -S(O)₂NRR' , -CN, -CF₃, -NO₂ and a phenyl ring having one or more substituents selected from the group consisting of halogen, nitro, cyano and trifluoromethyl; Z is a linking moiety selected from the group consisting of-C(=O)-, -S(O)-, -S(O)₂-, -S(O)₂NR-, wherein R and R' are each independently hydrogen, C₁-C₁₂ alkyl or aryl; and Q is a phosphodiester or phosphotriester linking group, to produce a derivatized solid support having attached 1,3-diol linking groups suitably protected with protecting groups;
(b) optionally removing a portion of the protecting groups from the derivatized solid support to provide a derivatized solid support having 1,3-diol linking groups with synthesis initiation sites; and
(c) coupling small ligand molecules to the synthesis initiation sites on the derivatized solid support to produce a solid support having attached small ligand molecules which are removable therefrom upon application of base.

The conditions used for attaching the 1,3-diol linking group to the solid support, removing protecting groups, and coupling small ligand molecules to the synthesis initiation sites are all standard synthetic procedures found in, for example, M.J. Gait, ed., OLIGONUCLEOTIDE SYNTHESIS - A PRACTICAL APPROACH, IRL Press, Oxford, 1984, incorporated herein by reference.

### IV. Labels for Enhanced Oligomer Detection

In still other aspects, the present invention provides labels which can be used for 3'-end or 5'-end labeling of an oligomer prepared on a solid support. For use in solid phase oligomer synthesis, the labels of the present invention will have the formula: 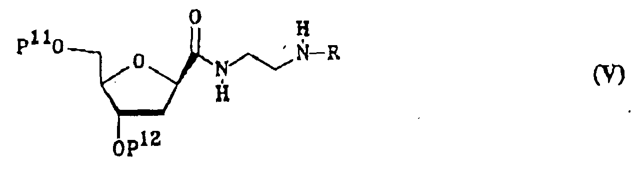 wherein P¹¹ and P¹² are each independently a hydrogen, a protecting group, a phosphoramidite, or trialkylammonium H-phosphonate and R is an fluorophore or group for label attachment (*e.g.,* biotin). In certain preferred embodiments P¹¹and P¹² are both hydrogen. In other preferred embodiment P¹¹ is a protecting group and P¹² is a phosphoramidite or trialkylammonium H-phosphonate. Preferred protecting groups are acid labile protecting groups, with DMT being particularly preferred.

A particularly preferred label has the formula: 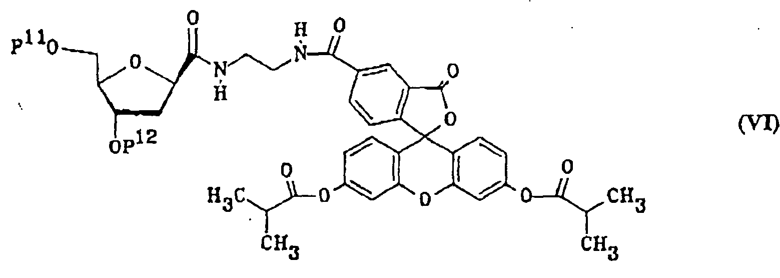 in which P¹¹and P¹² are defined as above.

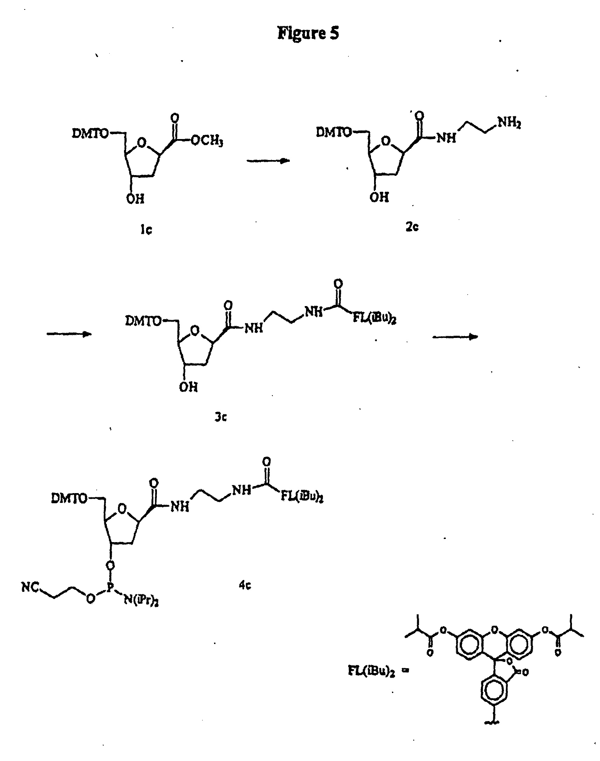

Preparation of these labels can be carried out essentially as depicted in Figure 5. According to the reaction scheme, DMT-protected allonic methyl ester 1c is treated with ethylene diamine to form the corresponding N-(2-aminoethyl) amide 2c. Reaction of the primary amine with isobutyryl-protected fluorescein N-hydroxysuccinimide ester provides a protected form of a fluorescein labeled modified sugar 3c. Conversion of the 3'-hydroxy group of the sugar to a phosphoramidite or other phosphodiester or phosphotriester-forming group can be accomplished using known reagents and conditions. Alternatively, the 3'-hydroxy group can be modified with other hydroxy protecting groups to provide a compound having greater synthetic flexibility. Suitable conditions (*e.g*., temperatures, time or reactions, concentration and solvent) are provided in the examples below, In a related aspect, the present invention provides a substrate for the solid phase synthesis of oligonucleotides. The modified substrate has the formula:

A¹¹-B¹¹-L¹¹-Fl

in which A¹¹ is a solid support, B¹¹ is a bond or a spacer, L¹¹ is a linking group, and Fl is a label having the formula: 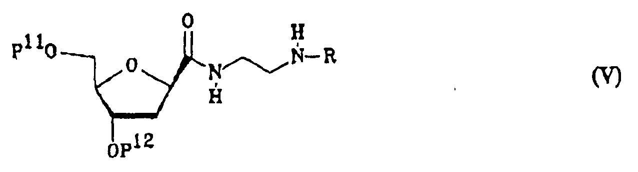 wherein P¹¹ and P¹² are each independently a hydrogen, a protecting group, a phosphoramidite, or trialkylammonium H-phosphonate and R is an fluorophore or group for label attachment (*e.g.,* biotin). Preferably Fl is a fluorescent moiety having the formula: 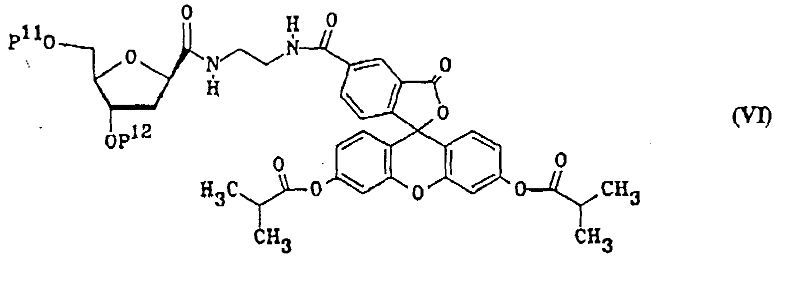 wherein one of P¹¹ and P¹² is a covalent bond to L¹¹ and the other of P¹¹ and P¹² is hydrogen, a protecting group, or a phosphoramidite.

In still other related aspects, the invention provides substrate bound fluorescently labeled oligonucleotides having the formulae:

A¹¹-B¹¹-L¹¹-Nu-Fl

or

A¹¹-B¹¹-L¹¹-Fl-Nu

In each of the above formulae, A¹¹ is a solid support, B¹¹ is a bond or a spacer, L¹¹ is a linking group, Nu is an oligonucleotide and Fl is as defined above. When Fl is at the terminus of the oligonucleotide it will have the formula: 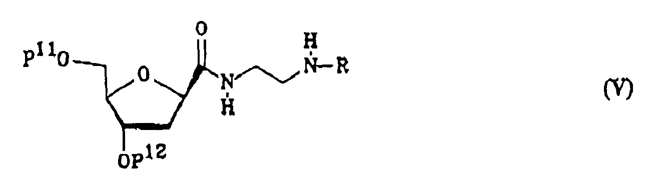 wherein one of P¹¹ and P¹² represents a bond and the other of P¹¹ and P¹² represents a hydrogen, protecting group, or phosphorus-ester forming group. The R group is as defined above. For those embodiments in which Fl is between L¹¹ and Nu, Fl will have the above formula in which P¹¹ and P¹² each represent bonds.

In each of these related aspects, the labeling group is preferably of the formula: 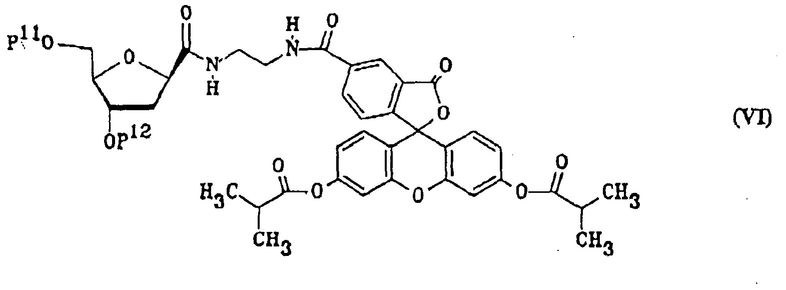 with P¹¹and P¹² having the meanings provided above. Additionally, standard solid phase techniques (including supports, solvents, temperatures and the like) are used for construction of the modified supports and attached oligomers.

### VI. EXAMPLES

### EXAMPLE 1

This example illustrates the synthesis of an unsymmetrical disulfide linking group having a DMT protecting group at one terminus and a phosphoramidite activating group at the other terminus.

### (a) Conversion of 6-bromohexanol to 6-bromohexyl allyl carbonate

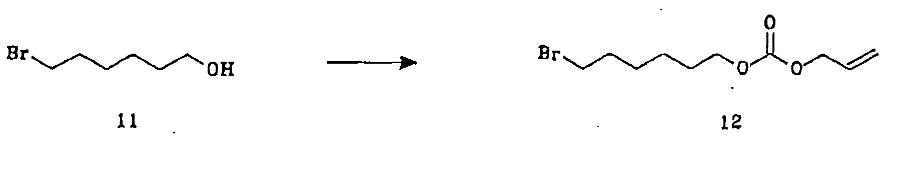

To a solution of 6-bromohexanol (4.8 g) in 20 mL of pyridine at 0°C to 20°C, was added allyl chloroformate (4.5 mL, 40 mmol). The resulting mixture was stirred at room temperature overnight. Diethyl ether was added and the mixture was washed sequentially with saturated NaHCO₃, water, and saturated NaCl. The organic layer was dried over anhydrous Na₂SO₄, filtered and evaporated under reduced pressure to provide crude 6-bromohexyl allyl carbonate 12 (3.627 g) as a colorless oil. Flash chromatography (hexane/Et₂O, 2/1 as eluant) provided the purified product 12 (2.158 g).

### (b) Preparation of 6-mercaptohexyl allyl carbonate

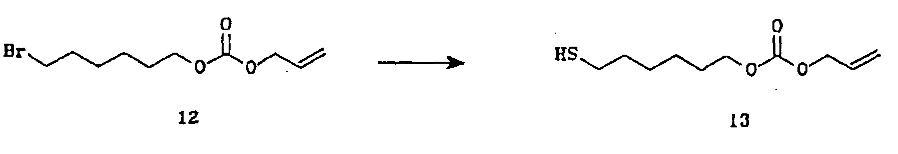

Sodium hydrogen sulfide (3.6 g) was dissolved in aqueous pH 7 buffer (10 mL) and 6-bromohexyl allyl carbonate (0.4 g) was added. Tetrahydrofuran (-15 mL) was added and the mixture was stirred at room temperature overnight. The mixture was diluted with 25 mL of diethylether. The organic layer was washed with water and brine and dried over anhydrous Na₂SO₄. The salt was removed by filtration and the solvent was removed from the filtrate to provide a crude product which was used in the next step without further purification.

### (c) Preparation of disulfide 14

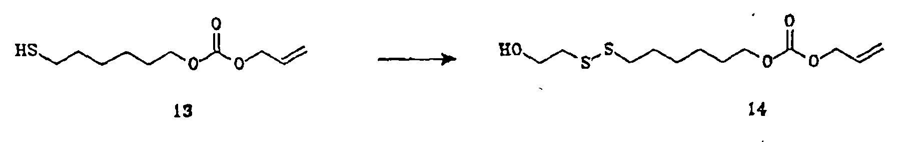

6-Mercaptohexyl allyl carbonate 13 (388 mg), 2-hydroxyethyl 2-pyridyl disulfide (350 mg) and triethylamine (1.0 mL) were combined in 5 mL of THF. The reaction was stirred for 1 hr. The mixture was concentrated under reduced pressure and the residue was purified by flash chromatography (silica; hexane/diethyl ether, 1/1) to provide the desired disulfide 14 (422 mg) as a clear colorless oil.

### (d) Preparation of Monoprotected Disulfide 16

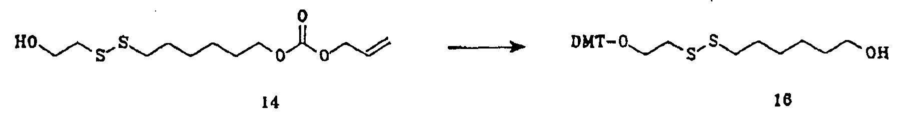

The hydroxy disulfide 14 (1.8 g, 6.1 mmol, 1.0 eq.) was combined with DMT-Cl (2.4 g, 6:7 mmol, 1,1 eq.) in 20 mL of dry pyridine under an atmosphere of argon. After 4 hr the mixture was diluted with ethyl acetate (50 mL) and poured into 50 mL of saturated aqueous NaHCO₃. The resulting mixture was extracted with EtOAc (50 mL), and the organic extract was washed with saturated aqueous NaCl and dried over anhydrous Na₂SO₄. Evaporation of the solvent provided the crude intermediate product 16 as an orange oil.

The oil was combined with 50 mL of 20% THF in anhydrous MeOH and a catalytic amount of anhydrous K₂CO₃ was added. The mixture was stirred at room temperature overnight. EtOAc (50 mL) was added and the resulting mixture was poured into 50 mL of saturated aqueous NaHCO₃. The layers were separated and the aqueous portion was extracted with two 50 mL portions of ethyl acetate, The combined organic portions were washed with saturated aqueous NaCl and dried over anhydrous Na₂SO₄. Evaporation of the solvent provided the crude product as an orange oil. Purification was carried out by flash chromatography (hexane/EtOAc, 3/7 with 1% triethylamine) to provide 2.65 g (73% for the two steps) of product 16 as a pale yellow oil.

### (e) Preparation of DMT-protected, Phosphoramidite (UDL) 17

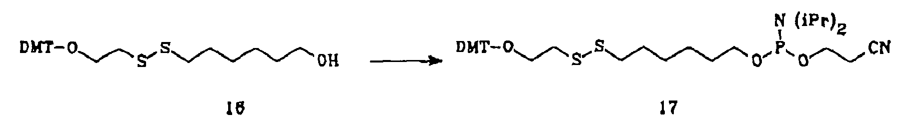

DMT-protected disulfide 16 (2.0 g, 3.35 mmol, 1.0 eq.) and DIPAT (2.87 g, 1.68 mmol, 0.5 eq.) were combined in 20 mL of dry CH₂Cl₂, under an atmosphere of argon. CEBAP (1.1 g, 1.2 mL, 3,69 mmol, 1.1 eq.) was added and the mixture was stirred at room temperature for 3 hr. The resulting mixture was poured into saturated aqueous NaHCO₃ and the organic layer was separated, washed with saturated aqueous NaCl and dried over Na₂SO₄. Removal of solvent under reduced pressure provided the DMT-protected, phosphoramidite 17 as a pale yellow oil. Purification by flash chromatography, eluting first with hexane containing 1% triethylamine, then with ethyl acetate/hexane (1/9, containing 1% triethylamine), provided 2.06 g (77%) of 17 as a pale yellow oil. ¹H NMR and ³¹P NMR were consistent with the assigned structure.

The unsymmetrical disulfide linking group can be used in any automated synthesizer under conditions which are useful for standard phosphoramidites. Typically, the molarity of the iodine solution used in these syntheses is reduced from 0.2 M to 0.02 M.

### EXAMPLE 2

This example illustrates the preparation of a fluorescein phosphoramide as outlined in Figure 5.

### (a) Conversion of Ester 21 to N-(2-aminoethyl)amide 22

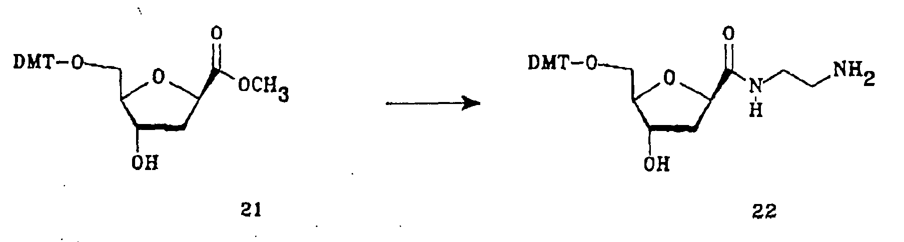

Ethylene diamine (5.6 mL, 84 mmol, 40 eq.) was combined with 10 mL of dry acetonitrile and cooled to 0°C under an atmosphere of argon. Ester 21 (1.0 g, 2.1 mmol, 1.0 eq.) was added and the resulting mixture was heated to reflux for 20 hr. The solvent was evaporated to leave a pale yellow oil which was dissolved in 25 mL of EtOAc and poured into 25 mL of saturated aqueous NaHCO₃. The organic layer was drawn off and the aqueous layer was extracted with EtOAc (2 × 25 mL). The combined organic portions were washed with saturated aqueous NaCl, dried over Na₂SO₄ and filtered. The solvent was removed under reduced pressure to provide 1.1 g of the amide 22 as a white foam (100% yield) which was carried on without additional purification.

### (b) Attachment of fluorescein label to provide 23

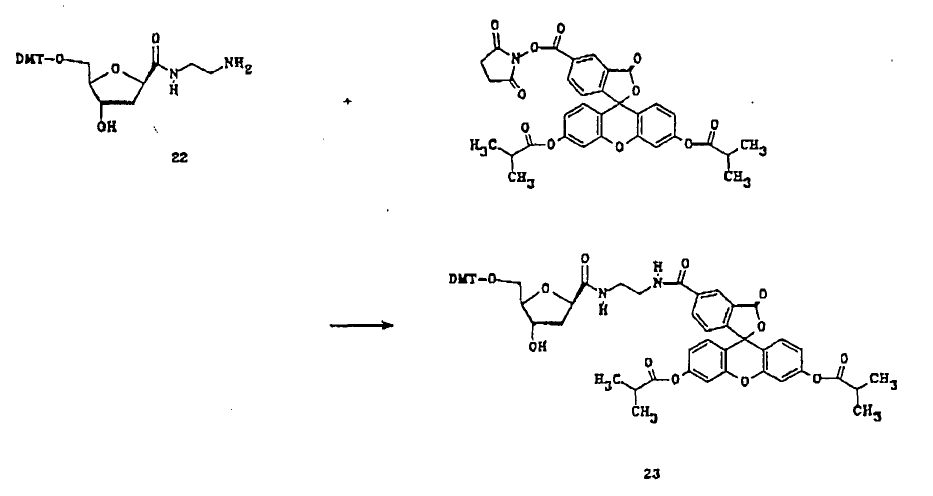

The N-(2-aminoethyl)amide 22 of step (a) (770 mg, 1.5 mmol, 1.0 eq.) was combined with triethylamine (418 µL, 3.0 mmol, 2.0 eq.) in 10 mL of dry THF and the mixture was cooled to 0°C and placed under an atomosphere of argon. A solution of diisobutyrl-protected, 5-carboxyfluorescein N-hydroxysuccinimide (920 mg, 1.5 mmol, 1.0 eq.) in 5 mL of dry THF was added and the mixture was stirred at 0°C for 30 min. The resulting mixture was diluted into 25 mL of EtOAc, washed twice with saturated aqueous NaHCO₃ (cold 1/10 diluted) followed by saturated aqueous NaCl. The organic layer was then dried over Na₂SO₄, filtered and evaporated to leave 1.9 g of a yellow foam.

The product was purified using flash chromatography (2 % MeOH in CH₂Cl₂ as eluant). Fractions containing the product were combined and solvent was removed under reduced pressure to provide the product 23 as a white foam. ¹H NMR was consistent with the assigned structure.

### (c) Conversion of 23 to phosphoramidate 24

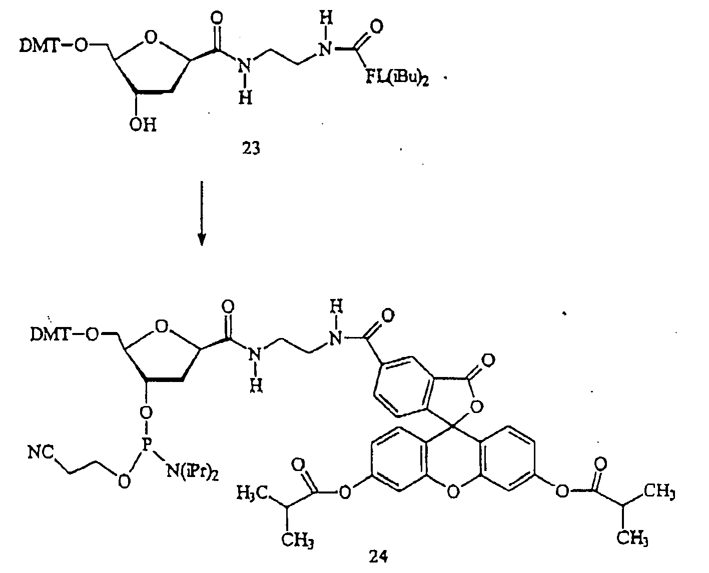

The product of 23 (500 mg, 0.5 mmol, 1.0 eq.) was combined with DIPAT (43 mg, 0.25 mmol, 0.5 eq.) in 5 mL of dry CH₂Cl₂ under an atmosphere of argon. CEBAP (174 µL, 0.55 mmol, 1.1 eq.) was added and the mixture was stirred at room temperature overnight. The resulting mixture was diluted into 25 mL of EtOAc, washed with saturated aqueous NaHCO₃ (cold 1/10 diluted, 2 × 25 mL) followed by saturated NaCl (25 mL). The organic layer was then dried over Na₂SO₄, filtered and evaporated to leave 800 mg of a yellow foam.

The product was purified using flash chromatography (20% hexane in CH₂Cl₂ with 1% triethylamine as eluant). Fractions containing the product were combined and solvent was removed under reduced pressure to provide 520 mg (87%) of the product 24 as a white foam.

The linking group 24 can be coupled to supports or oligomers under the same conditions used for any other standard nucleoside phosphoramidite in an automated synthesizer.

### VII. Conclusion

The above description is illustrative and not restrictive. Many variations of the invention will become apparent to those of skill in the art upon review of this disclosure. Merely by way of example a variety of reaction conditions, protecting groups and monomers may be used without departing from the scope of the invention. The scope of the invention should, therefore, be determined not with reference to the above description, but instead should be determined with reference to the appended claims along with their full scope of equivalents.

## Claims

1. A compound of the formula: 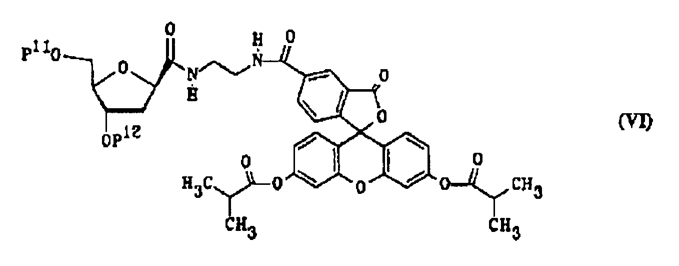 wherein P¹¹ and P¹² are each independently selected from the group consisting of hydrogen, a protecting group, and a phosphodiester-forming group.

2. A compound in accordance with claim 1, wherein P¹¹ and P¹² are both hydrogen.

3. A compound in accordance with claim 1, wherein P¹¹ is a protecting group and P¹² is a phosphoramidite.

4. A compound in accordance with claim 1, wherein P¹¹ is DMT and P¹² is a phosphoramidite.

5. A substrate for solid phase nucleic acid synthesis, said substrate having the formula:
A¹¹-B¹¹-L¹¹-Fl
wherein A¹¹ is a solid support, B¹¹ is a bond or a derivatizing group, L¹¹ is a linking group, and Fl is a fluorescent moiety having the formula: 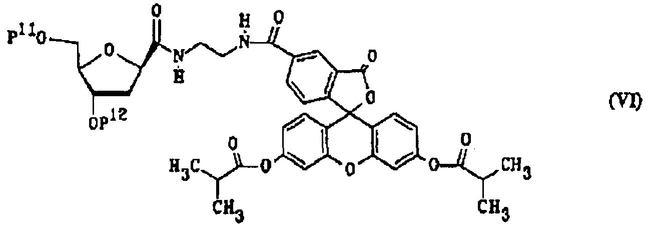 wherein one of P¹¹ and P¹² is a covalent bond to L¹¹ and the other of P¹¹ and P¹² is selected from the group consisting of hydrogen, a protecting group, and a phosphoramidite.

6. A substrate bound, fluorescently labeled nucleic acid having the formula:
A¹¹-B¹¹-L¹¹-Nu-Fl
wherein A¹¹ is a solid support, B¹¹ is a bond or a derivatizing group, L¹¹ is a linking group, Nu is a nucleic acid and Fl is a fluorescent moiety having the formula: 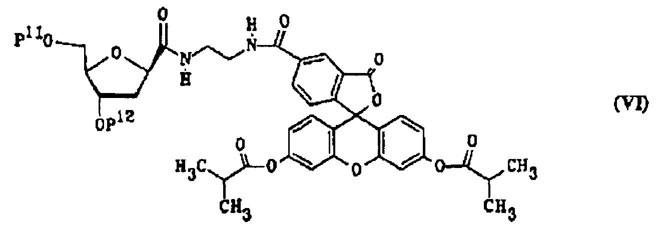 wherein one of P¹¹ and P¹² is a covalent bond to L¹¹ and the other of P¹¹ and P¹² is selected from the group consisting of hydrogen, a protecting group, and a phosphoramidite.

7. A substrate bound, fluorescently labeled nucleic acid having the formula:
A¹¹-B¹¹-L¹¹- Fl-NU
wherein A¹¹ is a solid support, B¹¹ is a bond or a derivatizing group, L¹¹ is a linking group, and F1 is a fluorescent moiety having the formula: 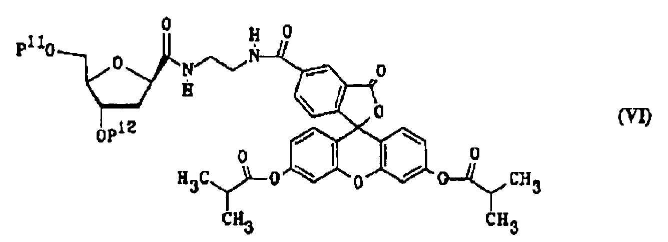 wherein each of P¹¹ and P¹² represents a bond; and Nu is a nucleic acid.

8. A selectively cleavable linkage molecule useful in solid phase compound synthesis, said linkage molecule having the formula: 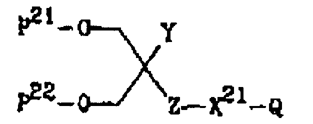 wherein
P²¹ and P²² are each protecting groups with the provisos that P²¹ can be removed under conditions which will not remove P²², and P²² can be removed under conditions which will not remove P²¹;
X²¹ is a linking moiety selected from the group consisting of an alkylene chain and an aryl group;
Y is a substituent selected from the group consisting of -C(=O)R, -S (O) R, -S(O)₂R, -S(O)₂NRR' , -CN, -CF₃, -NO₂ and a phenyl ring having one or more substituents selected from the group consisting of halogen, nitro, cyano and trifluoromethyl;
Z is a linking moiety selected from the group consisting of -C(=O-, -S(O)-, -S(O)₂, -S(O)₂NR-,
wherein
R and R' are each independently selected from the group consisting of hydrogen, C₁-C₁₂ alkyl and aryl; and
Q is a phosphate ester-forming group selected from the group consisting of a phosphoramidite and a trialkylammonium H-phosphonate.

9. A selectively cleavable linkage molecule in accordance with claim 8, wherei X²¹ is an amino alkoxy group, Y is -C(=O)R, Z is -C(O)- and Q is a phosphoramidite.

10. A selectively cleavable linkage molecule in accordance with claim 8, wherein P²¹ is removable under photolytic conditions, P²² is removable under acidic conditions, X²¹ is an amino alkoxy group, Y is -C(=O)R, Z is -C(O)- and Q is a phosphoramidite.

11. A selectively cleavable linkage molecule in accordance with claim 8, wherein P²¹ is MeNPOC, P²² is DMT, X²¹ is -NH-CH₂CH(CH₃)-O-, Y is -C(=O)R, Z is -C(O)- and Q is a phosphoramidite.

12. A modified substrate for use in solid phase chemical synthesis, said substrate having the formula:
L²¹- B²¹-A²¹
wherein A²¹ is a solid support, B²¹ is a bond or a derivatizing group, and L²¹ is a linking group having the formula: 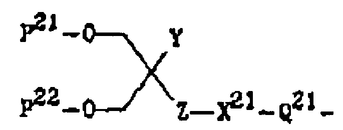 wherein
P²¹ and P²² are each protecting groups with the provisos that P²¹ can be removed under conditions which will not remove P²², and P²² can be removed under conditions which will not remove P²¹;
X²¹ is a linking moiety selected from the group consisting of an alkylene chain and an aryl group;
Y is a substituent selected from the group consisting of -C(=O)R, -S(O)R, -S(O)₂R, -S(O)₂NRR', -CN, -CF₃, -NO₂ and a phenyl ring having one or more substituents selected from the group consisting of halogen, nitro, cyano and trifluoromethyl;
Z is a linking moiety selected from the group consisting of -C(=O)-, -S(O)-, -S(O)₂, -S(O)₂NR-,
wherein
R and R' are each independently selected from the group consisting of hydrogen, C₁-C₁₂ alkyl and aryl; and
Q²¹ is a phosphate ester linking group.
